# EUROPEAN PATENT APPLICATION

(11) **EP 1 365 226 A2**
(43) Date of publication of application: **26.11.2003**
(21) Application number: 03011754.3
(22) Date of filing: 23.05.2003
(51) Int. Cl.: G01N 1/00, G01N 1/22, G01N 33/00

(54) **Gas introduction apparatus and gas analyser**

(30) Priority: 24.05.2002 JP 2002151236
(71) Applicant: Hitachi, Ltd., Chiyoda-ku, Tokyo 101-8010 (JP)
(72) Inventor: Fujita, Hiroyuki, Hitachi, Ltd., Intell. Property, Chiyoda-ku, Tokyo 100-8220 (JP); Iwasaki, Toshio, Hitachi, Ltd., Intell. Property, Chiyoda-ku, Tokyo 100-8220 (JP)
(74) Representative: Strehl Schübel-Hopf & Partner

(57) **Abstract**

Prior to initiation of gas analysis, a first vacuum valve (22) and a second vacuum valve (24) are closed while a third vacuum valve (30) is opened, then an introduction pipe (14) and a branch pipe (26) are evacuated by a vacuum pump (28), the third vacuum valve (30) is closed while the first vacuum valve (22) is opened so as to introduce and reserve gas to be analyzed into and in the gas introduction pipe when luggage (16) is set in a luggage receiver, and the second vacuum valve (24) is opened so as to feed the gas to be analyzed from the introduction pipe (14) into a mass spectrometer (12) for gas analysis.

## Description

### BACKGROUND OF THE INVENTION

The present invention relates to a gas introduction apparatus, and in particular to a gas introduction apparatus preferable for introducing gas to be analyzed, containing gaseous molecules having stuck to an object to be examined, and for feeding the same into a gas analyzer.

Conventionally, in order to analyze gas containing gaseous molecules having stuck to luggage or the like with the use of a gas analyzer (mass spectrometer) such as a quadrupole mass spectrometer or a gas chromatograph, there has been utilized such a configuration that the gas is introduced from a suction port of a luggage receiver into the gas analyzer through an introduction pipe by a pump incorporated in the gas analyzer, and then the thus introduced gas is analyzed in the analyzer. Further, as disclosed in the JP-A-2-159538, there has been proposed such a configuration that a suction pump is connected in a pipe line connecting a container receiving therein luggage with a gas extraction container in order to suck gas from the luggage.

In this case, the introduction pipe or the pipe line usually has a relatively long length up to several meters, and accordingly, with the configuration in which the gas is sucked up by the pump, it takes several seconds until the gas expelled from the luggage flows into the gas analyzer through the introduction pipe. Thus, it is difficult to instantly carry out gas spectrometry for the gas expelled from the luggage.

Thus, there has been proposed such a configuration that the introduction pipe is connected thereto with a bypass passage on the gas analyzer side, and a bypass valve is connected to the bypass passage in order to increase the flow rate of the gas flowing through the introduction pipe and to bypass a large part of the gas in the introduction pipe by way of the bypass valve while a small part of the gas in the introduction pipe is led into the gas analyzer. With this arrangement, the time required for the gas flowing through the introduction pipe is shortened in order to promote the mass spectrometry. It is noted that, for example, JP-A-5-506303 discloses the apparatus of this kind.

In a conventional configuration in which no bypass valve is incorporated in an introduction pipe, it takes a long time to discharge air inherently stagnating in the introduction pipe or to pass gas to be analyzed introduced into the introduction pipe, and accordingly, a start of the analysis is delayed by that time. Thus, the analysis of the gas in the mass spectrometer cannot be made at a high rate. Further, in order to enhance the analytical sensitiveness, it is preferable to use a highly sensitive mass spectrometer, but the higher the sensibility of the mass spectrometer, the lower the appropriate flow rate of gas flowing through an analyzing portion for analyzing the gas, it is difficult to aim at making both highly sensitive analysis and high speed analysis consistent with each other in such a configuration that an introduction pipe is connected with a suction pump.

Meanwhile, in the case of the provision of the bypass valve in the introduction pipe, the time by which gas passes through the introduction pipe can be shortened, but since the flow rate of gas led into the mass spectrometer is lower while the flow rate of gas discharged through the bypass valve is higher, thus gas is discharged wastefully. If the absolute value of a quantity of gas introduced into the introduction pipe is small, the flow rate of gas cannot be increased anyway, thereby it is difficult to increase the speed of the analysis. It is noted that the gas may be diluted with ambient air in order to ensure a required flow rate of the gas, but the gas diluted with the air hinders the analysis from increasing its sensibility.

### BRIEF SUMMARY OF THE INVENTION

An object of the present invention is to provide a gas introduction apparatus which can make both high speed gas analysis and highly sensitive gas analysis consistent with each other, and a gas analysis system using this gas introduction apparatus.

To the end, according to the present invention, there is provided a gas introduction apparatus comprising a gas introduction passage which introduces thereinto gas to be analyzed, containing gas molecules having stuck to an object to be examined, from the object side, for feeding therethrough the gas to a gas analyzer, an exhaust means connected to the gas introduction passage, for discharging the gas from the gas introduction pipe so as to evacuate the interior of the introduction pipe to a pressure lower than the atmospheric pressure, a first vacuum valve for opening and closing the gas introduction passage on the object side, a second vacuum valve for opening and closing the gas introduction pipe on the gas analyzer side, wherein prior to initiation of the gas analysis, the interior of the gas introduction passage is evacuated to a pressure lower than the atmospheric pressure by the exhaust means after the first vacuum valve and the second vacuum valve are closed, and thereafter, the first vacuum valve is opened so as to reserve the gas to be analyzed in the gas introduction pipe, then the first vacuum valve is closed while the second vacuum valve is opened in order to discharge the gas to be analyzed from the gas introduction pipe into the gas analyzer.

In the configuration of the gas introduction apparatus, there is provided a controller for controlling the opening and closing of the vacuum valves, with which the first and second vacuum valves are closed, prior to initiation of gas analysis by the gas analyzer, then the first vacuum valve is opened after the gas introduction passage is evacuated by the exhaust means to a pressure lower than the atmospheric pressure, in order to introduce and reserve the gas to be analyzed in the gas introduction passage, and thereafter, the first vacuum valve is closed while the second vacuum valve is opened in such a condition that the pressure in the gas introduction pipe becomes a predetermined value, in order to discharge the gas to be analyzed from the gas introduction pipe into the gas analyzer.

Further, in the configuration of the gas introduction apparatus, a vacuum pump connected to the gas introduction pipe, for evacuating the gas introduction pipe may be used, instead of the exhaust means.

Further, there may be provided a third vacuum valve which is connected in a branch passage connecting between the above-mentioned vacuum pump and the above-mentioned gas introduction passage so as to open and close the branch passage, and the vacuum valves may be controlled by the controller. Prior to initiation of gas analysis by the gas analyzer, the first vacuum valve and the second vacuum valve are closed while the third vacuum valve is opened, then the interior of the gas introduction passage is evacuated by the vacuum pump, and thereafter, the first vacuum valve is opened while third vacuum valve is closed so as to introduce gas to be analyzed into the gas introduction passage. In such a condition that the pressure in the gas introduction passage becomes a predetermined value, control is carried out in such a way that the first vacuum valve is closed while the second vacuum valve is opened so as to discharge the gas to be analyzed from the gas introduction passage into the gas analyzer.

The gas introduction apparatus may include the following configurations.

There are incorporated a vacuum container connected in the branch passage between the third vacuum valve and the gas introduction passage, for reserving the gas to be analyzed introduced in the branch passage from the gas introduction passage, and a fourth vacuum valve connected in the branch passage between the third vacuum valve and the vacuum container, for opening and closing a passage for introducing the atmospheric air into the branch passage, and the above-mentioned controller carries out such control that the fourth vacuum valve is closed until the pressure in the gas introduction passage becomes a predetermined value, and is then opened with such a condition that the pressure in the gas introduction passage becomes the predetermined value.

Moreover, the gas introduction apparatus may be composed of a gas introduction passage into which gas to be analyzed containing gas molecules having stuck to an object to be examined is introduced, and through which the gas to be analyzed is led into a gas analyzer, a compressor for compressing the gas to be analyzed introduced into the gas introduction passage from the objective side and for discharging the gas through the gas introduction passage to the gas analyzer side, a buffer container for reserving the gas to be analyzed discharged from the compressor, and a pressure regulator valve for reducing the pressure of the gas to be analyzed reserved in the buffer container and discharging the gas to be analyzed into the gas analyzer.

Further, the gas introduction apparatus may be composed of a gas introduction passage into which gas to be analyzed containing gas molecules having stuck to an object to be examined is introduced, and through which the gas to be analyzed is led into a gas analyzer, a variable volume buffer container for reserving the gas to be analyzed introduced from the gas introduction passage on the object side with its variable volume, and a pressure regulator valve for reducing the pressure of the gas to be analyzed reserved in the variable volume buffer container and discharging the gas into the gas analyzer.

Meanwhile, there may be provided such a configuration that a group of gas introduction apparatuses each having any one of the above-mentioned configurations, are provided for a single gas analyzer, wherein gas introduction passages in the gas introduction apparatuses are connected to the gas analyzer, and the controllers in the apparatuses carry out such control that gas to be analyzed is discharged from the gas introduction passages, successively into the gas analyzer.

Further, there may be provided such a configuration that a group of gas introduction apparatuses each having any of the above-configurations with no vacuum pump, are provided for a single gas analyzer, wherein gas introduction passages are connected to the gas analyzer, through the intermediary of a single vacuum pump used commonly in the group, and the controllers in the apparatuses carry out such control that gas to be analyzed is discharged from the gas introduction passages, successively into the gas analyzer.

Further, there is provided a gas analyzing system composed of a gas introduction apparatus having any one of the configuration as mentioned above, and a gas analyzer for analyzing masses in the gas to be analyzed discharged from the gas introduction apparatus so as to specify gas molecules contained the gas to be analyzed.

With the measures as stated above, since there is provided such a configuration that prior to initiation of gas analysis by the gas analyzer, the first vacuum valve and the second vacuum valve are closed, then the gas introduction passage is evacuated by the exhaust means or a vacuum pump to a pressure lower than the atmospheric pressure, and the first vacuum valve is closed but the second vacuum valve is opened in such a condition that the pressure in the gas introduction passage becomes a predetermined value in order to cause the gas to be analyzed to rush into the gas analyzer from the gas introduction passage. Thus, the gas to be analyzed can be introduced in to the gas analyzer by a required quantity with an increased flow rate per unit time without diluting the gas to be analyzed, thereby it is possible to make both high speed gas analysis and high sensitive gas analysis consistent with each other.

Other objects, features and advantages of the invention will become apparent from the following description of the embodiments of the invention taken in conjunction with the accompanying drawings.

### BRIEF DESCRIPTION OF SEVERAL VIEWS OF THE DRAWING

Fig. 1 is a block diagram illustrating a first embodiment of a gas analyzing system according to the present invention;
Fig. 2 is a block diagram illustrating a second embodiment of a gas analyzing system according to the present invention;
Fig. 3 is a block diagram illustrating a third embodiment of a gas analyzing system according to the present invention;
Fig. 4 is a block diagram illustrating a fourth embodiment of a gas analyzing system according to the present invention;
Fig. 5 is a block diagram illustrating a fifth embodiment of a gas analyzing system according to the present invention; and
Fig. 6 is a block diagram illustrating a sixth embodiment of a gas analyzing system according to the present invention.

### DESCRIPTION OF PREFERRED EMBODIMENTS OF THE INVENTION

### (First Embodiment)

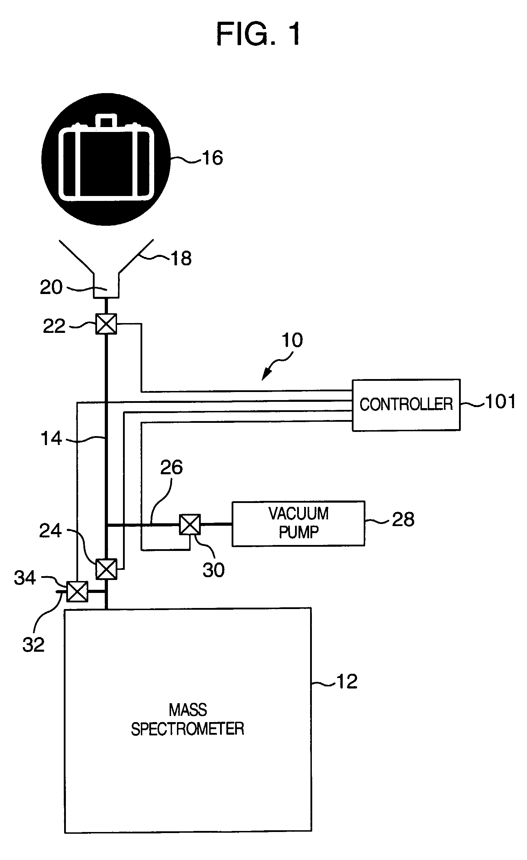

Explanation will be hereinbelow made of a first embodiment of a gas analyzing system according to the present invention with reference to Fig. 1. Referring to Fig. 1, the gas analyzing system is composed of a gas introduction apparatus 10 and a mass spectrometer 12.

The gas introduction apparatus 10 comprises a gas introduction pipe 14 which is connected on its inlet side with a suction port of a luggage receiver 18 for receiving luggage as an object to be examined, and which is connected on its outlet port side with the mass spectrometer 12. The gas to be analyzed containing the gas molecules such as nitro compound having a nitro group, having stuck to the luggage 16 is introduced through the gas introduction pipe 14 as a gas introduction and transfer passage and is led into the gas analyzer 12. The introduction gas pipe 14 is connected on the inlet side with a first vacuum valve 22 for opening and closing the inlet side of the gas introduction and transfer passage, and on the outlet side with a second vacuum valve 24 for opening and closing the outlet side of the gas introduction and transfer passage.

Further, a branch pipe 26 constituting a branch passage is connected to the introduction pipe 14 between the vacuum valves 22 and 24, and is connected on its outlet side with a vacuum pump 28 and in its intermediate part with a third vacuum valve 30 for opening and closing the branch pipe 26, that is, the branch passage. Further, a bypass pipe 32 constituting a bypass passage is connected to the introduction pipe 14 between the second vacuum valve 24 and the mass spectrometer 12, and is connected thereto with an auxiliary vacuum valve 34 for opening and closing the bypass pipe 32.

The vacuum pump 28 evacuates the introduction pipe 14 and the branch pipe 26 so as to lower the pressure in the introduction pipe 14 and the branch pipe to a value which is lower than the atmospheric air in order to introduce the gas to be analyzed thereinto.

Further, the introduction pipe 14 and the branch pipe 26 may be coated over their inner surfaces with an inactive material such as fluoride resin in order to prevent adsorption of the gas to be analyzed thereto. Meanwhile, with the use of such a configuration that the introduction pipe 14 and the branch pipe 26 are heated by a heater up to a temperature of about 100 to 300 deg.C, it is also possible to prevent the gas to be analyzed from adsorbing to the inner surfaces of the introduction pipes 14, 26.

Further, although the vacuum valves 22, 24, 30, 34 may be manually controlled, but they may be automatically controlled remotely by instructions from a controller 101.

Further, solenoid valves or pneumatically operated valves may be used as the vacuum valves 22, 24, 30, 34, and in order to control the opening and closing of the vacuum valves by the controller, operation timings of the vacuum values are set in view of a detection output from a pressure switch for detecting a pressure in the introduction pipe, or in view of a result of measurement or calculation of a time until the pressure of the gas in the introduction pipe 14 becomes a value with which the gas analysis can be made.

Next, the operation of the gas analyzing system having the above-mentioned configuration will be explained. Prior to initiation of gas to be analyzed containing gas molecules having stuck to the luggage 16, during a stand-by state period before the gas to be analyzed is introduced into the introduction pipe 14, the vacuum valves 22, 24 are closed while the vacuum valves 30, 34 are opened so that the introduction pipe 14 and the branch pipe 26 are evacuated through the operation of the vacuum pump 28. As the auxiliary valve 24 is opened at this time, fresh ambient air is introduced into a mass spectrometer 12.

Next, when the luggage 16 is set in the luggage receiver 18, the vacuum valve 30 is closed while the vacuum valve 22 is opened so as to cause the gas to be analyzed containing molecular particles having stuck to the luggage 16 to rush into the introduction pipe 14 and the branch pipe 26 through the suction port 20 and the vacuum valve 22. In this case, since the interiors of the introduction pipe 14 and the branch pipe 26 have been previously evacuated, the gas to be analyzed can be introduced thereinto at a remarkably high speed in comparison with such a case that air remains in the introduction pipe 14 and the branch pipe 26.

Thereafter, when the pressure in the introduction pipe 14 and the branch pipe 26 becomes a value at which the gas analysis can be made, the vacuum valve 24 is opened after the auxiliary vacuum valve 34, and the gas to be analyzed reserved in the introduction pipe 14 and the branch pipe 26 is discharged into the mass spectrometer 12 which therefore carries out mass spectrometry so as to determine whether the gas molecules contained in the gas to be analyzed are of a nitro compound or not. If the gas to be analyzed contains a nitro compound, such a fact that the nitro compound sticks to the luggage 15 is specified and outputted as a result of the gas analysis.

When the gas analysis by the mass spectrometer 12 is completed, the vacuum vales 22, 24 are closed while the vacuum valve 30 and the auxiliary vacuum valve 34 are opened, and through the operation of the vacuum pump 28, the introduction pipe 14 and the branch pipe 26 are evacuated, and then a stand-by state is set up until next analysis.

In this embodiment, since the introduction pipe 14 and the branch pipe 26 are evacuated beforehand, the gas to be analyzed can be introduced at a remarkably high speed in comparison with such a configuration that air remaines in the introduction pipe 14 and the branch pipe 26. For example, in the case of using a pipe having an inner diameter of 5 mm and a length of 10 m as the introduction pipe 14, when the gas to be analyzed is introduced into the mass spectrometer 12 at a flow rate of 0.5 l/min appropriate for gas analysis, the introduction of the gas to be analyzed takes a time which is longer than 20 sec until the gas to be analyzed has passed through the introduction pipe 14 if the introduction pipe 14 is not evacuated so that air is present therein. On the contrary, if the introduction pipe 14 has been previously evacuated, the introduction of the gas to be analyzed takes a time of about 0.1 sec.

Thus, in this embodiment, since the introduction pipe 14 and the branch pipe 26 are previously evacuated, the gas to be analyzed having a quantity required for the gas analysis can be introduced at a high speed by increasing a flow rate per unit time, thereby it is possible to aim at promoting the gas analysis in the mass spectrometer, and further, since the introduced gas to be analyzed can be discharged without being diluted into the mass spectrometer 12, thereby it is possible to aim at enhancing the sensitiveness of the gas analysis in the mass analyzer 12. Thus, according to this embodiment, both high speed and high sensitiveness of the gas analysis in the gas analyzer 12 can consist with each other.

### (Second Embodiment)

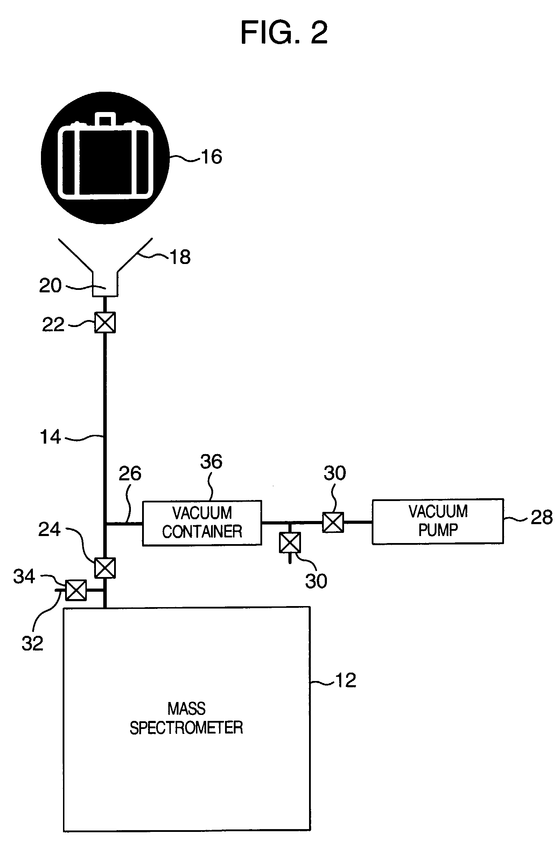

Next, explanation will be hereinbelow made of a second embodiment of the gas analyzing system according to the present invention with reference to Fig. 2. The configuration of this embodiment is the same as that of the first embodiment shown in Fig. 1, except that the a vacuum container 36 for reserving therein gas to be analyzed by a quantity required for gas analysis, is connected in the branch pipe 26, and a fourth vacuum valve 38 for opening and closing a passage for introducing the atmospheric air (the ambient air) is connected in the branch pipe 26 between the vacuum container 36 and the vacuum valve 30.

The vacuum valve 38 is opened and closed under the control of the controller, and is closed during a period until the gas to be analyzed is introduced into the introduction pipe 14 and the branch passage 26. Further, after the air remaining in the introduction pipe 14 and the branch pipe 26 is expelled by the vacuum pump 28 so as to evacuate the introduction pipe 14 and the branch pipe 26, the vacuum valve 22 is opened so that the gas to be analyzed is reserved in the vacuum container 36 by a quantity required for at least one time of the gas analysis. Thereafter, when the gas analysis is started by the mass spectrometer 12, the vacuum valve 30 is closed while the vacuum valve 38 is opened, the atmospheric air is introduced into the vacuum container 36. When the gas to be analyzed is expelled from the vacuum container 36 by the atmospheric air, the gas to be analyzed is discharged into the mass spectrometer 12 by way of the vacuum valve 24 without being diluted.

In this embodiment, similar to the first embodiment, the gas to be analyzed can be introduced by a quantity required for the gas analysis at a high speed, and the introduced gas to be analyzed can be discharged into the mass spectrometer 12 without being diluted. Thus, both high speed and high sensitiveness of the gas analysis in the mass spectrometer 12 can consist with each other.

Further, in this embodiment, since the gas can be introduced into the vacuum container by a quantity required for one time of gas analysis, the introduction of gas by a quantity required for the analysis can be facilitated, and the concentration of the gas to be analyzed can be prevented from being diluted due to intermixing of the air in the surrounding atmosphere, thereby it is possible to enhance the sensitiveness of the gas analysis.

Further, according to this embodiment, after the gas to be analyzed is introduced into the vacuum container 36, the luggage 39 can be removed, and accordingly, it can be replaced with a next luggage during a period in which the gas to be analyzed reserved in the vacuum container 36 is subjected to the gas analysis. Thereby it is possible to carry out gas analysis with a higher degree of efficiency in comparison with that of the previous embodiment.

### (Third Embodiment)

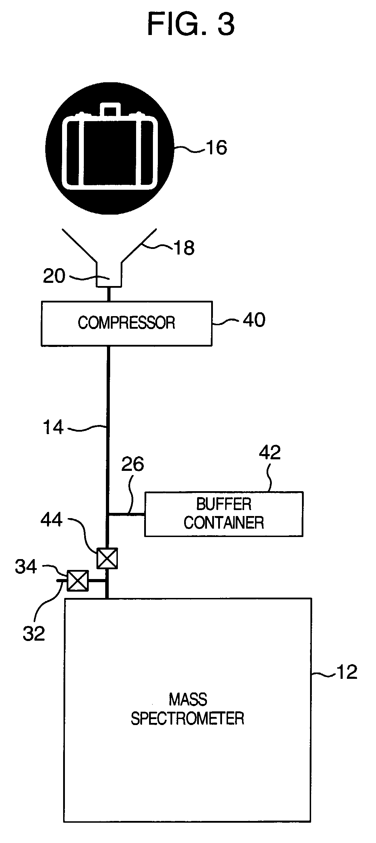

Next, explanation will be hereinbelow made of a third embodiment of the gas analyzing system according to the present invention with reference to Fig. 3. This configuration of this embodiment is the same as that of the first embodiment shown in Fig. 1, except that the vacuum valves 22, 24, 30 are removed, and instead thereof, the introduction pipe 14 is connected on the inlet side thereof to a compressor 40 and on its outlet side with a pressure regulator valve 44 while the branch pipe 26 is connected on the outlet side thereof with a buffer container 42.

The compressor 40 is configured to compress and feed the gas to be analyzed introduced from the suction port 20, to the outlet side, and the buffer container 42 is configured to reserve therein the gas to be analyzed fed from the compressor 40. The pressure regulator valve 44 is adapted to lower the pressure of the gas to be analyzed from the buffer container 42 and to introduce the mass spectrometer 12.

In this embodiment in which the gas to be analyzed introduced from the suction port 20 is pressurized in a large volume, and the thus compressed gas to be analyzed is fed into the buffer container 42, the large volume of the gas to be analyzed is reserved in the buffer container at a high speed. Further, the pressure of the gas to be analyzed reserved in the buffer container 42 is lowered to a value appropriate for the analysis thereof, that is, for example, a subatmospheric pressure, and then, the gas is introduced into the mass spectrometer 12.

Even in this embodiment, similar to the first embodiment, the gas to be analyzed is introduced into the mass spectrometer 12 at a high speed by a quantity required for the analysis thereof without being diluted, thereby both high speed and high sensitiveness of the gas analysis can consist with each other in the mass spectrometer 12.

### (Fourth Embodiment)

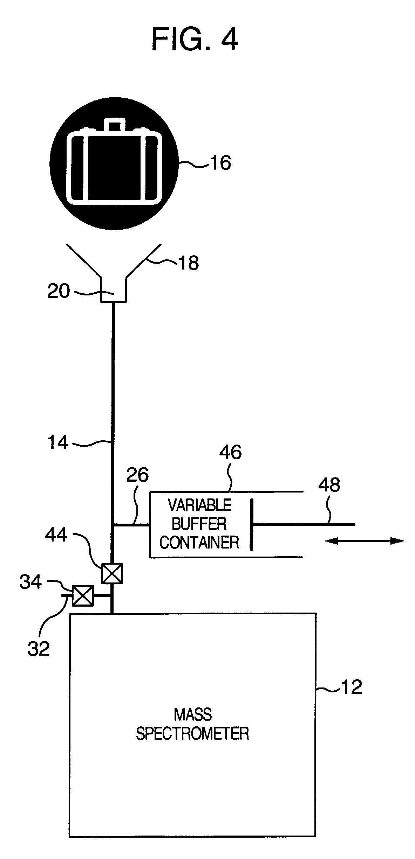

Next, explanation will be made of a fourth embodiment of the gas analyzing system according to the present invention with reference to Fig. 4. This embodiment is the same as that of the third embodiment shown in Fig. 3, except that the compressor 40 as shown in Fig. 3 is removed from the introduction pipe 14 while a variable volume buffer container 46 is connected to the branch pipe 26, instead of the buffer container 42.

The variable volume buffer container 46 is configured to reserve the gas to be analyzed introduced through the introduction pipe 16 and the branch pipe 26. That is, due to sucking action of a piston 48 which is reciprocally inserted in the variable volume buffer container 46, a negative pressure is effected in the container 46, the gas to be analyzed is introduced into the introduction pipe 14 and the branch pipe 26, and is then reserved therein. Meanwhile, due to compressing action of the piston 48, the gas to be analyzed reserved in the container 46 is compressed and discharged therefrom, and is then introduced into the mass spectrometer 12 in such a condition that the pressure of the gas is reduced to a subatmospheric pressure,

With this embodiment, in addition to the technical effects and advantages obtained in the third embodiment, it is possible to eliminate the necessity of the compressor 40 having a large capacity.

### (Fifth Embodiment)

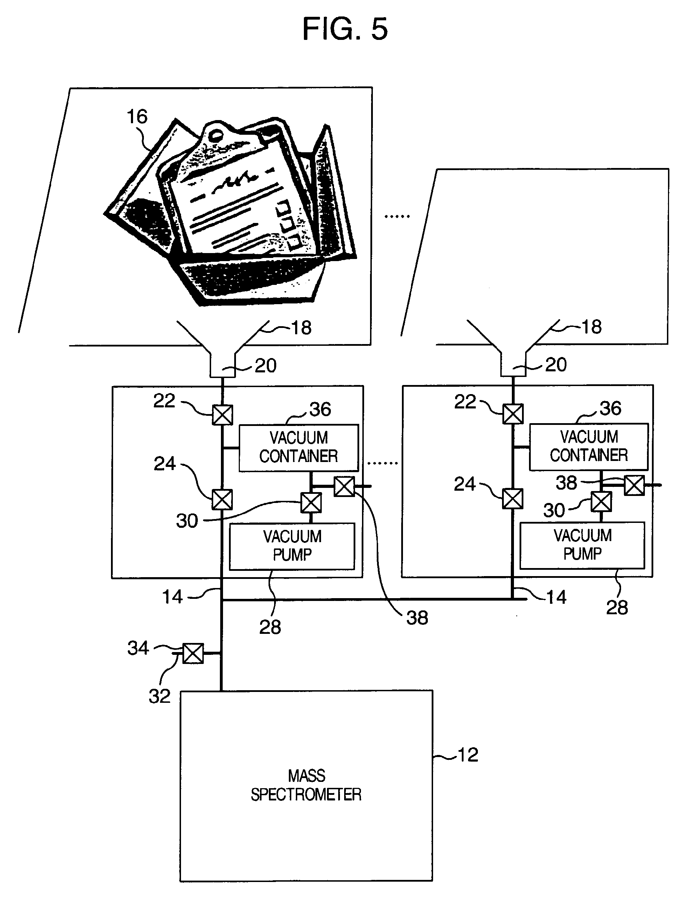

Next, explanation will be hereinbelow made of a fifth embodiment of the gas analyzing system according to the present invention with reference to Fig. 5. In this embodiment, a group of gas introduction apparatuses each similar to the gas introduction apparatus shown in Fig. 2 are provided to one mass spectrometer 12, and accordingly, the introduction pipes 14 in the respective apparatuses are connected to the mass spectrometer 12. In this configuration, the controllers in the apparatuses are adapted to carry out such control that the gas to be analyzed in the introduction pipes 14 in the respective apparatuses is successively introduced into the gas spectrometer 12. With this configuration, gas to be analyzed introduced by a plurality of gas introduction apparatuses 10 can be analyzed, sequentially with use of a single mass spectrometer 12.

Even in this embodiment, the gas to be analyzed can be introduced at a high speed by a quantity required for the analysis thereof, and the introduced gas to be analyzed can be fed into the mass spectrometer 12 without being diluted, thereby it is possible to allow both high speed and sensitiveness of the gas analysis to consist with each other. Further, the gas to be analyzed introduced through a plurality of gas introduction apparatuses 10 can be analyzed by a single mass spectrometer 12, sequentially.

### (Sixth Embodiment)

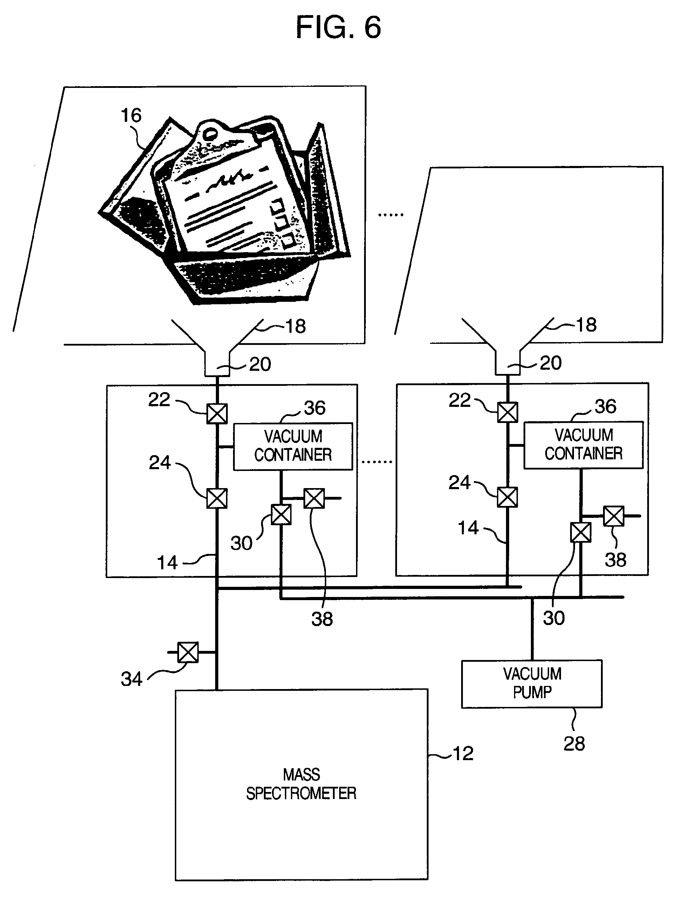

Explanation will be hereinbelow made of a sixth embodiment of the gas analyzing system according to the present invention with reference to Fig. 6. The configuration of this embodiment is the same as that of the fifth embodiment shown in Fig. 5, except that a single vacuum pump 28 is commonly used for a plurality of gas introduction apparatuses, instead of two vacuum pumps 28.

In this embodiment, in addition to technical effects and advantages similar to those obtained in the fifth embodiment, the configuration can be simplified in comparison with the configuration of the fifth embodiment since the vacuum pump 28 is commonly used.

Further, although the explanation has been made in the fifth and sixth embodiments such that each of the gas introduction apparatuses is that shown in Fig. 2, any of the gas introduction apparatuses shown in Figs. 1, 3 and 4 may be used therefor.

As stated above, according to the present invention, since gas to be analyzed is introduced by a quantity required for the analysis therefor after the pressure of the gas introduction and transfer passage is reduced to a value lower than the atmospheric pressure or after the gas introduction passage is evacuated, and since the introduced gas to be analyzed is fed into the mass spectrometer without being diluted, it is possible to allow high speed and sensitiveness of the gas analysis to consist with each other in the gas analyzer.

It should be further understood by those skilled in the art that although the foregoing description has been made on embodiments of the invention, the invention is not limited thereto and various changes and modifications may be made without departing from the spirit of the invention and the scope of the appended claims.

## Claims

1. A gas introduction apparatus comprising a gas introduction and transfer passage through which gas to be analyzed containing gas molecules having stuck to an object to be examined is introduced from the object side and is led into a gas analyzer; an exhausts means connected to the gas introduction passage, for evacuating the gas introduction passage to a pressure lower than the atmospheric pressure, a first vacuum valve for opening and closing the gas introduction and transfer passage on the object side; and a second vacuum valve for opening and closing the gas introduction and transfer passage on the gas analyzer side; wherein prior to initiation of gas analysis carried by the gas analyzer, the first vacuum valve and the second gas vacuum valve are closed while the pressure in the gas introduction and transfer passage is reduced to a value lower than the atmospheric air by the exhaust means, then the first vacuum valve is opened so as to introduce and reserve the gas to be analyzed into and in the gas introduction and transfer passage, and thereafter, the first vacuum valve is closed while the second vacuum valve is opened so as to feed the gas to be analyzed into the gas analyzer from the gas introduction and transfer passage before the gas analysis is carried out by the gas analyzer.

2. A gas introduction passage as set forth in claim 1, further comprising a controller for controlling the vacuum valves, wherein prior to the initiation of the gas analysis, the controller closes the first vacuum valve and the second vacuum valve, then opens the first vacuum valve after the pressure in the gas introduction and transfer passage is reduced by the exhaust means to a value lower than the atmospheric pressure, so as to introduce and reserve the gas to be analyzed into and in the gas introduction and transfer passage, and thereafter, closes the first vacuum valve while opens the second vacuum valve in order to feed the gas to be analyzed into the gas analyzer in such a condition that the pressure in the gas introduction and transfer passage becomes lower than the atmospheric pressure.

3. A gas introduction apparatus as set forth in claim 1, wherein the exhaust means is composed of a vacuum pump for evacuating the gas introduction and transfer passage.

4. A gas introduction apparatus as set forth in claim 3, further comprising a controller for controlling the opening and closing of the vacuum valves, wherein prior to initiation of gas analysis by the gas analysis, the controller closes the first vacuum valve and the second vacuum valve while the gas introduction and transfer passage is evacuated by the vacuum pump, then opens the first vacuum valve so as to introduce the gas to be analyzed into the gas introduction and transfer passage, and thereafter, closes the first vacuum valve while opens the second vacuum valve so as to feed the gas to be analyzed from the gas introduction and transfer passage into the gas analyzer in such a condition that the pressure in the gas introduction and transfer passage becomes a predetermined value.

5. A gas introduction apparatus as set forth in claim 4, further comprising a third vacuum valve incorporated in a branch passage connecting the vacuum pump and the gas introduction and transfer passage, wherein prior to the initiation of gas analysis, the controller closes the first vacuum valve and the second vacuum valve but opens the third vacuum valve while the gas introduction and transfer passage is evacuated by the vacuum pump, then opens the first vacuum valve while closes the third vacuum valve so as to introduce the gas to be analyzed into the gas introduction and transfer passage, and thereafter closes the first vacuum valve but opens the second vacuum valve so as to feed the gas to be analyzed from the gas introduction and transfer passage into the gas analyzer in such a condition that the pressure in the gas introduction and transfer passage becomes a predetermined value.

6. A gas introduction apparatus as set forth in claim 5, further comprising a vacuum container connected in the branch passage between the third vacuum valve and the gas introduction and transfer passage, for reserving therein the gas to be analyzed introduced into the branch passage from the gas introduction and transfer passage, and a fourth vacuum valve connected in the branch passage between the third vacuum valve and the vacuum container, for opening and closing a passage through which the atmospheric air is introduced into the branch passage, wherein the controller closes the fourth vacuum valve until the pressure in the gas introduction passage becomes a predetermined value, and opens the fourth vacuum valve in such a condition that the pressure in the gas introduction passage becomes the predetermined value.

7. A gas introduction apparatus comprising a gas introduction and transfer passage through which gas to be analyzed containing gas molecules having stuck to an object to be examined is introduced from the object side to a gas analyzer, a compressor for compressing the gas to be analyzed introduced in the gas introduction and transfer passage from the object side and for discharging the gas to be analyzed through the introduction and transfer passage toward the gas analyzer side, a buffer container for reserving the gas to be analyzed discharged from the compressor, and a pressure regulator valve for reducing the pressure of the gas to be analyzed reserved in the buffer container, and feeding the gas to be analyzed into the gas analyzer.

8. A gas introduction apparatus comprising a gas introduction and transfer passage through which gas to be analyzed containing gas molecules having stuck to an object to be examined, is introduced from the object side to a gas analyzer, a variable volume buffer container for reserving the gas to be analyzed introduced through the gas introduction and transfer passage from the object side with a variable volume, and a pressure regulator valve for reducing the pressure of the gas to be analyzed reserved in the variable volume buffer container, and feeding the gas to be analyzed into the gas analyzer.

9. A gas introduction apparatus as set forth in claim 4, wherein a plurality of gas introduction apparatuses are provided for a single gas analyzer, the gas introduction and transfer passages in the gas introduction apparatuses are connected to the gas analyzer, and the controllers in the gas introduction apparatuses carry out such control that the gas to be analyzed is fed into the gas analyzer successively from the gas introduction and transfer passages in the gas introduction apparatuses.

10. A gas introduction apparatus comprising a plurality of gas introduction apparatuses provided for a single gas analyzer, each of the gas introduction apparatus including the components as set forth in claim 4, except the vacuum pump, and a common vacuum pump used for the gas introduction apparatuses, wherein the gas introduction and transfer passages in the gas introduction apparatuses are connected to the gas analyzer, and the controllers in the gas introduction apparatuses carry out such control that the gas to be analyzed is fed into the gas analyzer successively from the gas introduction and transfer passages in the gas introduction apparatuses.

11. A gas analyzing system comprising a gas introduction apparatus as set forth in any one of claims 4, 8, 9 and 10, and a gas analyzer for carrying out gas mass spectrometry for the gas to be analyzed fed from the gas introduction apparatus so as to specify gas molecules contained in the gas to be analyzed.
